# FASCICULE DE BREVET EUROPEEN

(11) **EP 1 343 424 B1**
(45) Date de publication et mention de la délivrance du brevet: **15.09.2004**
(21) Numéro de dépôt: 01994926.2
(22) Date de dépôt: 20.12.2001
(51) Int. Cl.: A61B 17/70

(54) **IMPLANT INTERVERTEBRAL A CALE DEFORMABLE**
ZWISCHENWIRBELIMPLANTAT MIT VERFORMBAREM KEIL
INTERVERTEBRAL IMPLANT WITH DEFORMABLE WEDGE

(30) Priorité: 22.12.2000 FR 0016857
(43) Date de publication de la demande: 17.09.2003
(73) Titulaire: Spine Next, La Cité Mondiale, 33000 Bordeaux (FR)
(72) Inventeur: SENEGAS, Jacques, F-33700 Merignac (FR); PASQUET, Denis, F-Pessac 33600 (FR); LE COUEDIC, Régis, F-33000 Bordeaux (FR)
(74) Mandataire: Dronne, Guy
(86) Numéro de dépôt international: PCT/FR2001/004094
(87) Numéro de publication internationale: WO 2002/051326

(56) Documents cités:
- WO-A-97/09000
- WO-A-99/40866
- FR-A- 2 722 088
- FR-A- 2 775 183

## Description

La présente invention concerne un implant intervertébral comportant une cale destinée à s'appliquer entre deux apophyses épineuses, ladite cale présentant deux gorges opposées définies chacune par deux ailes, les axes desdites deux gorges étant sensiblement parallèles entre eux et lesdites apophyses épineuses venant en appui dans lesdites deux gorges.

Des implants intervertébraux comprenant une cale apte à être insérée entre les apophyses épineuses, qui prolongent la partie postérieure des vertèbres, pour limiter le rapprochement de deux vertèbres consécutives l'une par rapport à l'autre sont bien connus.

En effet, les pathologies dégénératives du disque intervertébral conduisent à un rapprochement des vertèbres les unes par rapport aux autres pouvant aller jusqu'au contact, ce qui peut provoquer le pincement des racines nerveuses qui débouchent latéralement entre les vertèbres. Afin de remédier à cet inconvénient, on fixe, avec des moyens de fixation appropriés, au moins une cale entre les apophyses épineuses des deux vertèbres consécutives susceptibles d'entrer en contact lors du mouvement du rachis. De la sorte, la cale bloque le déplacement des vertèbres lorsqu'elles se rapprochent l'une de l'autre, notamment lors de l'extension du rachis.

Ces cales sont réalisées dans un alliage biocompatible rigide, généralement à base de titane, et sont indéformables sous l'action des efforts qui s'exercent sur elles contrairement au disque intervertébral normalement constitué qui lui est élastiquement déformable dans certaines limites. Ainsi, ces cales permettent d'éviter le contact entre deux vertèbres, mais elles suppléent de façon incomplète le disque intervertébral qui permet une mobilité relative des vertèbres.

Afin de réaliser des cales élastiquement déformables, il a été imaginé de réaliser des cales présentant deux éléments opposés en matériaux rigides formant gorges dans lesquelles prennent appui les apophyses épineuses de deux vertèbres, les deux éléments formant gorges étant reliés entre eux par deux parties de ressort à lame élastiquement déformable. Ainsi, la cale est globalement élastiquement déformable ce qui permet d'obtenir des propriétés aptes à reproduire les conditions physiologiques normales de déplacement relatif des vertèbres.

Cependant, la réalisation de ce type de cale est relativement complexe et les matériaux des ressorts à lame ne sont généralement pas biocompatibles. En outre, le ressort doit présenter une taille importante pour obtenir l'élasticité nécessaire et il occupe donc une grande portion d'espace.

Ainsi, il a été envisagé de réaliser, de manière plus aisée, des cales en une seule pièce dans un matériau rigide obtenu par polymérisation et présentant un module d'élasticité bien inférieur au module d'élasticité du titane. Le document FR 2,775,183, sur lequel est basé le préambule de la revendication 1, décrit une telle cale. Cependant, le module d'élasticité du matériau doit être suffisant pour que les gorges desdites cales puissent coincer correctement les apophyses épineuses et de ce fait, bien qu'elles soient plus déformables que les cales en alliages de titane, elles ne le sont pas suffisamment pour remplir leur fonction.

L'invention vise à proposer un implant intervertébral comportant une cale déformable réalisée dans un matériau rigide obtenu par polymérisation.

Ce but est atteint, selon l'invention, grâce au fait que la cale présente au moins un évidement central entre lesdites deux gorges, ledit évidement central traversant ladite cale de part en part selon un axe sensiblement parallèle auxdits axes desdites gorges, et le volume dudit évidemment central par rapport au volume total de ladite cale est compris entre 0,1 et 0,3 par quoi ladite cale est élastiquement déformable.

Ainsi, une caractéristique de l'invention réside dans la réalisation d'un évidement central dans une cale pleine relativement indéformable de façon à obtenir des parois mobiles les unes par rapport aux autres pour que la cale puisse se déformer élastiquement sans qu'elle soit fragilisée de façon trop importante. A la base de chaque gorge la paroi est apte à se déformer élastiquement sous contrainte, notamment lorsque les apophyses épineuses se rapprochent l'une de l'autre, puisque l'espace entre lesdites parois est libre.

De la sorte, la cale constitue un obstacle au rapprochement des vertèbres les unes par rapport aux autres. Cependant, les forces exercées par la cale sur les apophyses épineuses sont proportionnelles aux déplacements relatifs des deux vertèbres, puisque la cale est élastiquement déformable, ce qui permet de recréer des conditions physiologiques normales ou quasi normales du déplacement relatif des vertèbres.

Selon un premier mode particulier de réalisation de l'invention, l'évidement présente une forme sensiblement parallélépipédique rectangle dont deux faces parallèles sont perpendiculaires à un axe coupant perpendiculairement les axes desdites gorges.

Ainsi, le fond des deux gorges est constitué par une paroi, les deux parois des deux gorges étant sensiblement parallèles entre elles et perpendiculaires à la direction principale de rapprochement des deux vertèbres. De la sorte, la cale présente une amplitude de déformation importante, en compression notamment.

Selon un deuxième mode particulier de réalisation de l'invention, la cale comprend deux évidements superposés entre lesdites deux gorges, les axes desdits évidements et les axes desdites deux gorges étant sensiblement parallèles entre eux.

Selon ce mode particulier de réalisation, les deux évidements permettent d'obtenir trois cloisons aptes à se déformer élastiquement sous l'effet d'une contrainte normale auxdites cloisons.

Selon un mode préféré de réalisation de l'invention, l'implant intervertébral comprend en outre, au moins une bande de fixation apte à maintenir ladite cale entre lesdites apophyses et ladite cale présente : des moyens de fixation pour relier ladite bande à ladite cale ; et, des moyens de fixation auto-bloquants ménagés dans ses parois latérales, lesdits moyens de fixation auto-bloquants étant aptes à recevoir ladite bande de fixation pour la bloquer par rapport à ladite cale.

De la sorte, la bande de fixation est montée au préalable sur la cale avant l'intervention chirurgicale et l'opérateur peut insérer la cale entre les apophyses épineuses, puis la solidariser à ces apophyses en tirant à l'extrémité libre de la bande qui se coince dans les moyens de fixation auto-bloquants.

De manière avantageuse, les moyens de fixation sont formés dans ladite cale par au moins un perçage latéral d'une desdites ailes débouchant dans l'une desdites gorges. Ainsi, l'extrémité de la bande opposée à ladite extrémité libre est reliée à ladite cale en formant une boucle passant à travers le perçage latéral. La bande est fixée sur elle-même avec des moyens formant agrafe. Bien évidemment, le perçage latéral est uniquement destiné à fixer l'extrémité de la bande et il est distinct dudit évidemment central qui lui, est exclusivement destiné à conférer à la cale sa déformabilité.

Avantageusement, les moyens de fixation auto-bloquants sont formés dans ladite cale par au moins deux perçages latéraux distincts borgnes présentant une intersection et un perçage longitudinal traversant l'aile opposée à l'aile présentant un perçage latéral et débouchant dans ladite intersection, ledit perçage longitudinale et lesdits perçages latéraux étant aptes à recevoir ladite bande de fixation pour former une boucle.

Ainsi, l'extrémité libre de la bande de fixation est l'abord insérée dans le premier perçage longitudinal pour débouché dans ladite intersection, traverse l'un des deux perçages pour ressortir de la cale et être réinsérée pour déboucher à nouveau dans ladite intersection, puis ressort en sens inverse par ledit perçage longitudinal de façon à venir en frottements contre la portion de bande déjà introduite. De la sorte, la bande est immobilisée par rapport à la cale.

Selon un mode de réalisation préférentiel, l'implant conforme à l'invention comprend une seule bande de fixation dont la première extrémité est reliée à ladite cale par lesdits moyens de fixation et dont la seconde extrémité est reliée à ladite cale par les moyens de fixation auto-bloquants ménagés dans l'une de ses parois latérales ; et ladite cale présente, dans la paroi latérale opposée à ladite paroi latérale présentant les moyens de fixation auto-bloquants, des moyens de guidage dans lesquels ladite bande de fixation est apte à coulisser de façon que ladite bande soit apte à entourer lesdites apophyses épineuses et ladite cale.

Ainsi, la première extrémité de la bande est reliée à la cale au niveau du perçage latéral puis entoure l'apophyse épineuse qui est insérée dans la première gorge et rejoint la paroi latérale présentant les moyens de guidage. Ensuite, la bande entoure l'apophyse épineuse qui est insérée dans la deuxième gorge et rejoint les moyens de fixation auto-bloquants de façon à être immobilisée par rapport à la cale.

De façon particulièrement avantageuse la cale est réalisée par moulage en une seule pièce dans un matériau obtenu par polymérisation. De la sorte, les cales, selon l'invention, sont susceptibles d'être produites à des coûts avantageux au regard des cales obtenues par usinage.

Selon un mode particulier de mise en oeuvre de l'invention la cale est en polyéther-éther-cétone.

D'autres particularités et avantages de l'invention ressortiront à la lecture de la description faite ci-après de modes de réalisation particuliers de l'invention, donnés à titre indicatif mais non limitatif, en référence aux dessins annexés sur lesquels :
- la Figure 1 est une vue schématique en perspective montrant la cale, selon un premier mode particulier de réalisation, insérée entre les apophyses épineuses de deux vertèbres consécutives ;
- la Figure 2, est une vue schématique selon une section verticale de la cale conformément à la Figure 1 ; et,
- la Figure 3 est une vue schématique en perspective montrant la cale, selon un deuxième mode de réalisation particulier, insérée entre les deux apophyses épineuses de deux vertèbres consécutives.

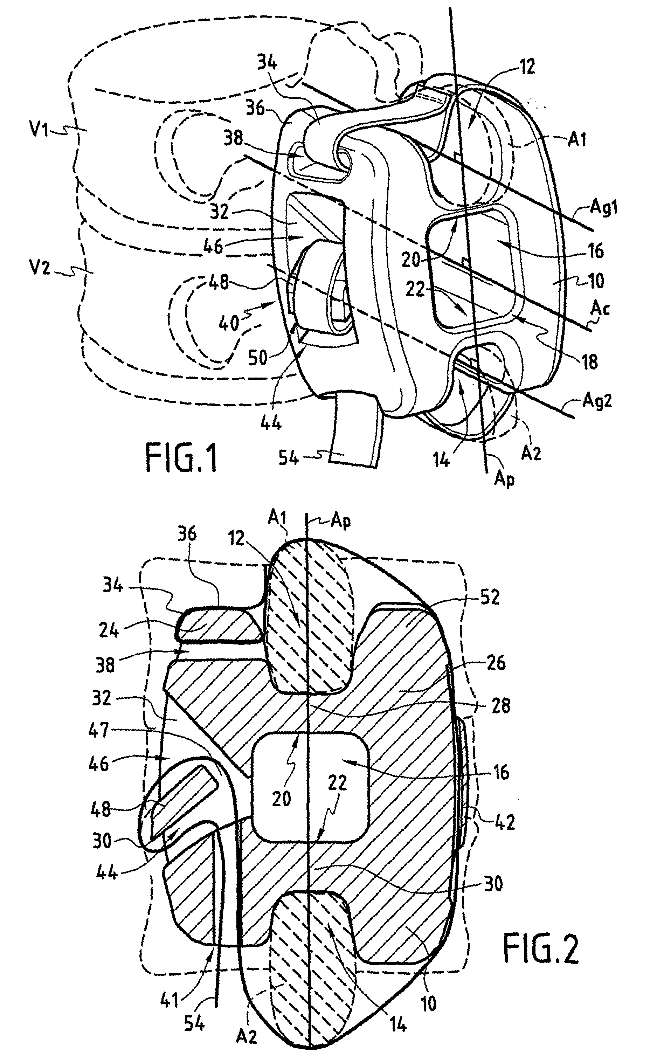

En se référant tout d'abord à la Figure 1, on décrira l'implant intervertébral conforme à l'invention selon un premier mode de réalisation.

On a représenté sur la Figure 1 deux vertèbres consécutives V1 et V2 respectivement prolongées, dans leur partie postérieure, par leur apophyse épineuse A1 et A2. Une cale 10, présentant une gorge supérieure 12 et une gorge inférieure 14 est insérée entre les apophyses épineuses A1 et A2 de façon que l'apophyse A1 soit en appui dans la gorge 12 et que l'apophyse A2 soit en appui dans la gorge 14. Les gorges supérieure 12 et inférieure14 présentent respectivement un axe Ag1 et Ag2 sensiblement parallèles entre eux.

La cale 10 présente un évidement central 16 situé entre les deux gorges 12 et 14, présentant une forme sensiblement parallélépipédique rectangle et traversant ladite cale 10 de part en part. Pour des raisons pratiques de réalisation de ladite cale 10 les coins 18 de l'évidement forme un arrondi sur toute l'épaisseur de la cale.

Par ailleurs, l'évidement central présente un axe Ac sensiblement parallèle aux deux axes Ag1 et Ag2 des deux gorges supérieure 12 et inférieure 14.

En outre, l'évidement central 16 présente une face supérieure 20 parallèle à une face inférieure 22 en regard, lesdites faces 20, 22 étant sensiblement perpendiculaires à un axe Ap coupant perpendiculairement les axes Ag1 et Ag2 desdites gorges 12 et 14, et l'axe Ac de l'évidement central.

La Figure 2 illustre la cale 10 en vue arrière, dans laquelle on retrouve l'axe Ap et les faces supérieure 20 et inférieure 22 qui lui sont perpendiculaires.

La cale 10 est réalisée par moulage dans un matériau du type polymère. Cependant, elle pourrait être obtenue par usinage en partant d'un bloc dudit matériau. Les matériaux utilisés pour réaliser les cales conformes à l'invention sont biocompatibles.

Avantageusement, on réalise la cale 10 conforme à l'invention en polyéther-éther-cétone. Ce matériau présente intrinsèquement un coefficient de déformation sous contrainte peu important et donc un haut module d'élasticité de l'ordre de 3,5 GPa. Cependant, lorsque la cale 10 est réalisée dans ce matériau et qu'un évidement central est pratiqué, elle se déforme plus aisément.

En outre, l'évidement représente entre 10 et 30% du volume de la cale. Avantageusement, la largeur de l'évidement central 16 représente 38% de la largeur totale de la cale 10 et sa hauteur représente 40% de la hauteur totale de la cale 10.

Lorsque l'évidement 16 représente 30% du volume de la cale 10, elle se déforme de façon importante lorsqu'un effort lui est appliqué sans que sa résistance mécanique ne soit compromise. En revanche, lorsque l'évidement 16 représente 10% du volume de la cale 10, elle se déforme peu mais avec une plus grande amplitude qu'une cale ne présentant pas d'évidement.

Ainsi, il est possible d'ajuster les capacités de déformation de la cale 10 en fonction des applications spécifiques auxquelles on la destine en pratiquant un évidement de taille appropriée.

Comme l'illustre la Figure 2, lorsque l'évidement central 16 est pratiqué, la cale 10 est divisée en deux portions, 24 et 26, les deux portions étant reliées par une paroi supérieure 28 et une paroi inférieure 30. Les parois supérieure 28 et inférieure 30 constituent respectivement le fond des deux gorges 12 et 14. On comprend que ces parois 28, 30 sont minces relativement à la portion de cale 10 qui sépare les deux gorges 12 et 14 lorsque la cale 10 ne présente pas d'évidement 16 et qu'elles sont susceptibles d'être flexibles par rapport auxdites portions 24 et 26.

De la sorte, les apophyses épineuses A1 et A2, lorsqu'elles se rapprochent l'une de l'autre, lors de l'extension du rachis, tendent à comprimer les deux parois 28 et 30 et à les entraîner également l'une vers l'autre. Bien évidemment, compte tenu de la présence de l'évidement, malgré le haut module d'élasticité du matériau, les deux parois 28 et 30 sont aptes à se déplacer élastiquement l'une vers l'autre et donc à induire une plus grande amplitude de mouvement des deux apophyses A1 et A2 l'une par rapport à l'autre.

Cet effort mécanique qui s'exerce en compression sur la cale provoque la déformation de l'évidement de façon à rapprocher les deux parois 28 et 30 l'une vers l'autre. Cependant, la déformation du rachis peut se faire également dans d'autres directions comme on le décrira plus en détails dans la suite de la description.

Afin de décrire les autres déformations possibles de la cale 10, on décrira le mode de fixation de la cale 10 sur les apophyses épineuses A1 et A2 comme l'illustre les Figures 1 et 2.

L'implant intervertébral conforme à l'invention et selon ce mode particulier de réalisation comprend une seule bande de fixation 32 qui permet de solidariser la cale 10 et les apophyses épineuses A1 et A2.

La première extrémité 34 de la bande 32 est reliée à la première aile 36 de la gorge supérieure 12 au moyen d'un perçage latérale 38 qui est pratiqué dans l'aile 36 et qui débouche dans la gorge supérieure 12. Ainsi, la première extrémité 34 de la bande 32 est insérée dans le perçage 38 de façon à former une boucle autour de la portion supérieure de l'aile 36 et est fixé sur elle-même avec des moyens d'agrafage. De la sorte, la bande 32 est reliée solidement à la cale 10 de façon à permettre la mise sous tension de la bande 32 sans dégradation de cette liaison.

La bande 32 entoure les apophyses épineuses A1 et A2 et la cale 10 de manière à les maintenir dans les gorges 12 et 14. Afin de fixer la bande 32 et de la guider sur la cale 10, cette dernière présente des moyens de fixation auto-bloquants 40 ménagés dans une paroi latérale et un perçage longitudinal 41 ainsi que des moyens de guidage 42 ménagés dans la paroi latérale opposée.

Les moyens de fixation auto-bloquants 40 sont formés par un premier perçage latéral 44 et un deuxième perçage latéral 46, qui se rejoigne dans une intersection 47 située dans l'épaisseur de la cale. En outre un perçage longitudinale 41 traverse l'aile de la gorge et débouche dans ladite intersection 47.

Par ailleurs, la portion de cale 48 séparant les deux perçages latéraux 44 et 46 présente un bord transversal saillant 50 apte à constituer des moyens de frottement et de blocage de la bande 32.

De la sorte, la bande 32 dont la première extrémité 34 est reliée à l'aile 36 prend appui sur la partie supérieure de l'apophyse épineuse A1 puis sur l'extrémité de la seconde aile 52 de la gorge supérieure 12 et s'insère dans les moyens de guidage 42 situés sur la paroi latérale de la cale 10. La bande 32 s'appuie ensuite sur l'apophyse épineuse A2 et sur les extrémités des deux ailes de la gorge inférieure 14. L'extrémité libre 54 de la bande 32 s'insère dans le perçage longitudinal 41 et traverse ladite intersection 47 pour ressortir de la cale par le deuxième perçage 46, puis la bande est insérée dans le premier perçage 44, traverse à nouveau ladite intersection 47 pour ressortir par le perçage longitudinal 41. De la sorte, la bande 32 est susceptible d'être bloquée en translation par frottement sur elle même dans le perçage longitudinal 41 lorsqu'elle est mise en tension. De plus, le bord saillant 50 de la portion de cale 48 permet un blocage de la bande 32.

Ainsi, la cale 10 est apte à être solidarisée aux apophyses épineuses A1 et A2 par la mise sous tension de la bande 32 qui est bloquée par rapport à la cale 10 dans les moyens auto-bloquants 40.

De la sorte, le déplacement des apophyses épineuses A1 et A2 l'une par rapport à l'autre, respectivement prisonnières des gorges 12 et 14, entraîne une déformation élastique de la cale 10 quelle que soit la direction de déplacement.

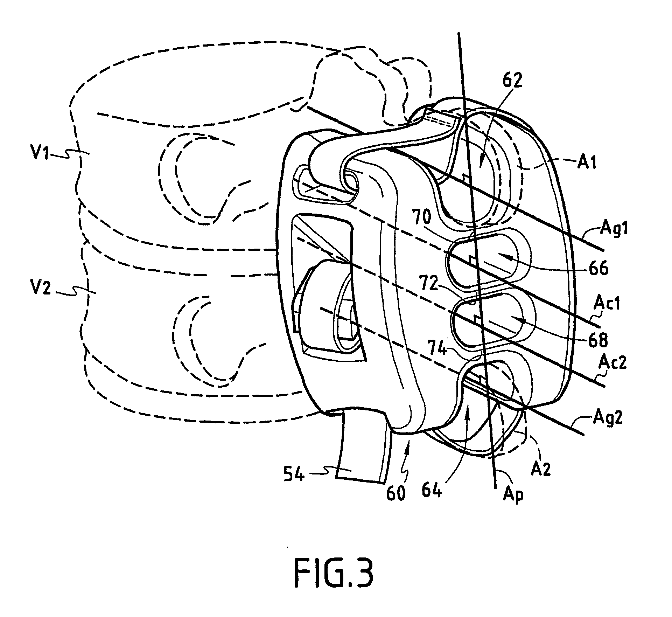

On se référera maintenant à la Figure 3 pour décrire un implant intervertébral selon un deuxième mode de réalisation où la cale présente deux évidements.

On retrouve sur la Figure 3 une cale 60 entre les deux vertèbres V1 et V2 lesquelles sont prolongées par leurs apophyses épineuses respectives A1 et A2 qui sont en appui dans des gorges supérieure 62 et inférieure 64 de la cale 60. Cette dernière est reliée aux apophyses épineuses au moyen d'une bande de fixation de façon analogue à la cale 10 représentée sur les Figures 1 et 2.

La cale 60 présente deux évidements superposés 66 et 68 d'axe respectif Ac1 et Ac2 sensiblement parallèles entre eux et aux axes Ag1 et Ag2 desdites gorges 62 et 64. Les évidements superposés 66 et 68 présentent une forme comportant deux faces superposées parallèles entre elles et deux parois courbes en regard.

Ainsi, la cale 60 est divisée en deux parties reliées entre elles par trois cloisons 70, 72 et 74 aptes à se déformer lorsqu'un effort est exercé sur la cale 60. Bien évidement, les capacités de déformation de la cale 60 sont moins importantes que celle de la cale 10 représentée sur les Figures 1 et 2 compte tenu du fait que les deux parties da la cale 10 sont reliées par deux cloisons seulement. Ceci est vrai uniquement si les cloisons 70, 72, 74 et 28, 30 sont sensiblement de même taille.

Dans ce deuxième mode particulier de réalisation de l'invention, les moyens auto-bloquants sont également réalisés en pratiquant deux perçages dans l'épaisseur de la paroi latérale de la cale 60, qui se rejoignent dans une intersection, et un perçage longitudinal pratiqué dans l'aile qui débouche dans ladite intersection.

Les deux modes de réalisation précédents permettent de réaliser des implants intervertébraux comportant des cales avec des propriétés mécaniques, notamment de déformations, adaptées aux réactions que l'on souhaite exercer sur les apophyses épineuses lorsque ces dernières se déplacent les unes par rapport aux autres.

L'invention n'est pas limitée aux modes de réalisation précédents, mais s'étend aux cales présentant au moins un évidement dans leur partie centrale quelle que soit la forme dudit évidement. En particulier, l'évidement est susceptible de présenter une forme circulaire.

## Revendications

1. Implant intervertébral comportant une cale destinée à s'appliquer entre deux apophyses épineuses, ladite cale présentant deux gorges opposées définies chacune par deux ailes, les axes desdites deux gorges étant sensiblement parallèles entre eux et lesdites apophyses épineuses venant en appui dans lesdites deux gorges,
**caractérisé en ce que** ladite cale (10) comprend au moins un évidement central (16) entre lesdites deux gorges (12, 14), ledit évidement central (16) traversant ladite cale (10) de part en part selon un axe Ac sensiblement parallèle aux axes Ag1 et Ag2 desdites gorges et **en ce que** le volume dudit évidemment central (16, 66, 68) par rapport au volume total de ladite cale (10) est compris entre 0,1 et 0,3, par quoi ladite cale (10) est élastiquement déformable.

2. Implant intervertébral selon la revendication 1, **caractérisé en ce que** ledit évidement (16) présente une forme sensiblement parallélépipédique rectangle dont deux faces parallèles (20, 22) sont perpendiculaires à un axe Ap coupant perpendiculairement les axes Ag1 et Ag2 desdites gorges.

3. Implant intervertébral selon la revendication 1, **caractérisé en ce que** ladite cale (10) comprend deux évidements superposés (66, 68) entre lesdites deux gorges (62, 64), les axes Ac1 et Ac2 desdits évidements et les axes Ag1 et Ag2 desdites deux gorges étant sensiblement parallèles entre eux.

4. Implant intervertébral selon l'une quelconque des revendications 1 à 3,
**caractérisé en ce qu'**il comprend en outre, au moins une bande (32) de fixation apte à maintenir ladite cale (10) entre lesdites apophyses;
et **en ce que** ladite cale (10) présente :
- des moyens de fixation (38) pour relier ladite bande à ladite cale ; et,
- des moyens de fixation auto-bloquants (40) ménagés dans ses parois latérales, lesdits moyens de fixation auto-bloquants étant aptes à recevoir ladite bande de fixation pour la bloquer par rapport à ladite cale.

5. Implant intervertébral selon la revendication 4, **caractérisé en ce que** lesdits moyens de fixation (38) sont formés dans ladite cale par au moins un perçage latérale (38) d'une desdites ailes débouchant dans l'une desdites gorges (12, 14, 62, 64).

6. Implant intervertébral selon la revendication 5, **caractérisé en ce que** lesdits moyens de fixation auto-bloquants (40) sont formés dans ladite cale (10) par au moins deux perçages latéraux distincts (46, 44) borgnes présentant une intersection (47) et un perçage longitudinal (41) traversant l'aile opposée à l'aile présentant un perçage latéral et débouchant dans ladite intersection (47), ledit perçage longitudinale (41) et lesdits perçages latéraux (44, 46) étant aptes à recevoir ladite bande de fixation (32) pour former une boucle.

7. Implant intervertébral selon la revendication 3 ou 4,
**caractérisé en ce qu'**il comprend une seule bande de fixation (32) dont la première extrémité (34) est reliée à ladite cale (10) par lesdits moyens de fixation (38) et dont la seconde extrémité (54) est reliée à ladite cale (10) par les moyens de fixation auto-bloquants (40) ménagés dans une de ses parois latérales;
et **en ce que** ladite cale (10) présente, dans la paroi latérale opposée à ladite paroi latérale présentant les moyens de fixation auto-bloquants (40), des moyens de guidage (42) dans lesquels ladite bande (32) de fixation est apte à coulisser de façon que ladite bande (32) soit apte à entourer lesdites apophyses épineuses A1 et A2 et ladite cale (10).

8. Implant intervertébral selon l'une quelconque des revendications 1 à 7, **caractérisé en ce que** ladite cale (10) est réalisée par moulage en une seule pièce d'un matériau obtenu par polymérisation.

9. Implant intervertébral selon l'une quelconque des revendications 1 à 9, **caractérisé en ce que** ladite cale (10) est en polyéther-éther-cétone.

## Patentansprüche

1. Zwischenwirbelimplantat, das einen Keil umfasst, der dazu bestimmt ist, sich zwischen zwei Gelenkfortsätze zu fügen, wobei der Keil zwei einander gegenüberliegende Hohlkehlen aufweist, die jede von zwei Flügeln definiert werden, wobei die Achsen der zwei Hohlkehlen im Wesentlichen zueinander parallel sind und wobei die Gelenkfortsätze in den zwei Hohlkehlen zum Aufliegen kommen,
**dadurch gekennzeichnet, dass** der Keil (10) mindestens eine zentrale Aussparung (16) zwischen den zwei Hohlkehlen (12, 14) aufweist, wobei die zentrale Aussparung (16) den Keil (10) entlang einer Achse Ac ganz durchquert, die im Wesentlichen zu den Achsen Ag1 und Ag2 der Hohlkehlen parallel ist, und dadurch, dass das Volumen der zentralen Aussparung (16, 66, 68) im Vergleich zum Gesamtvolumen des Keils (10) zwischen 0,1 und 0,3 liegt, wodurch der Keil (10) elastisch verformbar ist.

2. Zwischenwirbelimplantat nach Anspruch 1, **dadurch gekennzeichnet, dass** die Aussparung (16) eine im Wesentlichen parallelepipedische rechteckige Form aufweist, von der zwei parallele Flächen (20, 22) senkrecht zu einer Achse Ap stehen, die die Achsen Ag1 und Ag2 der Hohlkehlen senkrecht schneidet.

3. Zwischenwirbelimplantat nach Anspruch 1, **dadurch gekennzeichnet, dass** der Keil (10) zwei übereinander liegende Aussparungen (66, 68) zwischen den zwei Hohlkehlen (62, 64) umfasst, wobei die Achsen Ac1 und Ac2 der Aussparungen und die Achsen Ag1 und Ag2 der zwei Hohlkehlen im Wesentlichen zueinander parallel sind.

4. Zwischenwirbelimplantat nach einem der Ansprüche 1 bis 3,
**dadurch gekennzeichnet, dass** es ferner mindestens ein Befestigungsband (32) umfasst, das den Keil (10) zwischen den Gelenkfortsätzen halten kann; und dadurch, dass der Keil (10) Folgendes aufweist:
- Befestigungsmittel (38), um das Band mit dem Keil (10) zu verbinden; und
- selbstblockierende Befestigungsmittel (40), die in seinen Seitenwänden eingerichtet sind, wobei die selbstblockierenden Befestigungsmittel das Befestigungsband aufnehmen können, um es zum Keil zu blockieren.

5. Zwischenwirbelimplantat nach Anspruch 4, **dadurch gekennzeichnet, dass** die Befestigungsmittel (38) in dem Keil (10) von mindestens einer seitlichen Bohrung (38) eines der Flügel gebildet sind, die in eine der Hohlkehlen (12, 14, 62, 64) münden.

6. Zwischenwirbelimplantat nach Anspruch 5, **dadurch gekennzeichnet, dass** die selbstblockierenden Befestigungsmittel (40) in dem Keil (10) aus mindestens zwei getrennten seitlichen Sackbohrungen (46, 44) gebildet sind, die einen Schnittpunkt (47) aufweisen, und eine Längsbohrung (41), die den Flügel durchquert, der dem Flügel gegenüberliegt, der eine seitliche Bohrung aufweist und in dem Schnittpunkt (47) mündet, wobei die Längsbohrung (41) und die seitlichen Bohrungen (44, 46) das Befestigungsband (32) aufnehmen können, um eine Schleife zu bilden.

7. Zwischenwirbelimplantat nach Anspruch 3 oder 4,
**dadurch gekennzeichnet, dass** es ein einziges Befestigungsband (32) umfasst, dessen erstes Ende (34) mit dem Keil (10) durch die Befestigungsmittel (38) verbunden ist, und dessen zweites Ende (54) mit dem Keil (10) durch selbstblockierende Befestigungsmittel (40) verbunden ist, die in einer seiner Seitenwände eingerichtet sind;
und dadurch, dass der Keil (10) in der Seitenwand, die der Seitenwand gegenüber liegt, die die selbstblockierenden Befestigungsmittel (40) umfasst, Führungsmittel (42) aufweist, in welchen das Befestigungsband (32) gleiten kann, so dass das Befestigungsband (32) die Gelenkfortsätze A1 und A2 und den Keil (10) umgeben kann.

8. Zwischenwirbelimplantat nach einem der Ansprüche 1 bis 7, **dadurch gekennzeichnet, dass** der Keil (10) durch Formen aus einem einzigen Stück eines Werkstoffs hergestellt wird, der durch Polymerisation erzielt wird.

9. Zwischenwirbelimplantat nach einem der Ansprüche 1 bis 9, **dadurch gekennzeichnet, dass** der Keil (10) aus Polyäther-Äther-Keton besteht.

## Claims

1. An intervertebral implant including a wedge which is adapted to be inserted between two spinous processes, said wedge having two opposite grooves each being defined by two flanges, the axes of said two grooves being substantially parallel to each other and said spinous processes being engaged therein,
said implant being **characterised in that** said wedge (10) has at least one central opening (16) between said two grooves (12, 14), said central opening (16) passing completely through said wedge (10) along an axis Ac substantially parallel to the axes Ag1 and Ag2 of said grooves, and **in that** the volume of said central opening (16, 66, 68) is from 10% to 30% of the total volume of said wedge (10), which renders said wedge (10) elastically deformable.

2. An intervertebral implant according to claim 1, **characterised in that** said opening (16) has a substantially rectangular parallelepiped shape with two parallel faces (20, 22) perpendicular to an axis Ap intersecting the axes Ag1 and Ag2 of said grooves at right angles.

3. An intervertebral implant according to claim 1, **characterised in that** said wedge (10) has two superposed openings (66, 68) between said two grooves (62, 64), with the axes Ac1 and Ac2 of said openings and the axes Ag1 and Ag2 of said two grooves being substantially parallel to each other.

4. An intervertebral implant according to any one of claims 1 to 3,
**characterised in that** it further includes at least one fixing band (32) adapted to retain said wedge (10) between said processes; and
**in that** said wedge (10) has:
- fixing means (38) for connecting said band to said wedge; and
- self-locking fixing means (40) formed in its lateral walls, with said self-locking fixing means being adapted to receive said fixing band in order to immobilize it relative to said wedge.

5. An intervertebral implant according to claim 4, **characterised in that** said fixing means (38) are formed in said wedge by at least one lateral bore (38) in one of said flanges opening into one of said grooves (12, 14, 62, 64).

6. An intervertebral implant according to claim 5, **characterised in that** said self-locking fixing means (40) are formed in said wedge (10) by at least two separate blind lateral bores (46, 44) having an intersection (47) and a longitudinal bore (41) passing through the flange opposite the flange having a lateral bore and opening into said intersection (47), said longitudinal bore (41) and said lateral bores (44, 46) being adapted to receive said fixing band (32) to form a loop.

7. An intervertebral implant according to claim 3 or 4,
**characterised in that** it includes a single fixing band (32) whose first end (32) is connected to said wedge (10) by said fixing means (38) and whose second end (54) is connected to said wedge (10) by the self-locking fixing means (40) formed in one of its lateral walls,
and **in that** said wedge (10) has, in the lateral wall opposite said lateral wall having the self locking fixing means (40), guide means (42) in which said fixing band (32) can slide so that said band (32) is able to surround said spinous processes A1 and A2 and said wedge (10).

8. An intervertebral implant according to any one of claims 1 to 7, **characterised in that** said wedge (10) is made in one piece of a material obtained by polymerisation.

9. An intervertebral implant according to any one of claims 1 to 9, **characterised in that** said wedge (10) is made of polyether ether ketone.
